# EUROPEAN PATENT SPECIFICATION

(11) **EP 1 686 993 B1**
(45) Date of publication and mention of the grant of the patent: **12.01.2011**
(21) Application number: 04816942.9
(22) Date of filing: 03.11.2004
(51) Int. Cl.: A61K 31/44

(54) **VIRUCIDAL ACTIVITIES OF A COMPOSITION COMPRISING CETYLPYRIDINIUM CHLORIDE AND CITRIC ACID**
VIRUZIDE WIRKUNGEN VON EINER ZUBEREITUNG ENTHALTEND CETYLPYRIDINIUMCHLORID UND ZITRONENSÄURE
ACTIVITES VIROCIDES D'UNE COMPOSITION COMPRENANT DU CHLORURE DE CETYLPYRIDINIUM ET DE L'ACIDE CITRIQUE

(30) Priority: 07.11.2003 US 703969; 10.09.2004 US 939307
(43) Date of publication of application: 09.08.2006
(73) Proprietor: Viratox, L.L.C., Little Rock, AR 72201 (US)
(72) Inventor: CAPPS, Charles, L., Little Rock, AR 72212 (US)
(74) Representative: Bradley, Adrian
(86) International application number: PCT/US2004/036949
(87) International publication number: WO 2005/046738

(56) References cited:
- EP-A- 0 338 515
- EP-A- 0 355 536
- DE-A1- 2 429 033
- DE-A1- 19 823 319
- US-B2- 6 635 676
- ECKHOFF B ET AL: "VIRUCIDAL EFFICACY OF MOUTH AND THROAT ANTISEPTICS IN SUSPENSION ASSAY" HYGIENE + MEDIZIN, vol. 11, no. 9, 1986, pages 324-326, XP009113051 ISSN: 0172-3790
- LEWIS ET AL: "5039688 Method of preventing aids transmission resulting from blood transfusions" TRANSFUSION SCIENCE, PERGAMON PRESS, OXFORD, GB, vol. 13, no. 4, 1 October 1992 (1992-10-01), page ii, XP022645951 ISSN: 0955-3886 [retrieved on 1992-10-01]

## Description

### REFERENCE TO RELATED APPLICATIONS

This application is a continuation-in-part of U.S. Patent Application No. 10703,969, and claims priority thereto.

### BACKGROUND OF THE INVENTION

### 1. Field of the Invention

The present invention relates to the use of a quaternary ammonium compound as a virucidal agent for surface contact disinfection and for use in the medical treatment and prevention of infection caused by viral particles. Specifically, the present invention relates to the use of cetylpyridinium chloride in a low concentration solution for contact destruction of viral particles in a relatively short time period with subsequent residual associated toxicity to viral particles. More specifically, the present invention describes the composition and application of cetylpyridinium chloride from a dry composition powder to a solution for surface disinfection; as a spray or aerosol inhalant for respiratory treatment for the destruction or inhibition of the corona virus; and as a foam, film, liquid, or suppository in the prevention of HIV transmission.

### 2. Description of the Related Art

Viruses are considered to be the smallest infectious agents capable of replicating in living cells. The precise mechanism of nucleic acid transfer to the host cell and the subsequent function of the host to promote production of additional progeny is not fully understood. Viruses have a core of nucleic acid surrounded by a protein coat or coats and may be furthered enveloped as in the case of the corona virus. The nucleic acids are of two types, ribose (RNA) or deoxyribose (DNA). This genetic material is responsible for directing replication once a suitable host is invaded. Without a protein coat, the nucleic acid is normally incapable of entering a cell.

In general, the DNA viruses multiply in the nucleus of the host cell, whereas the RNA viruses multiply in the cytoplasm. Some RNA viruses appear to emerge as buds from the cell membrane. Generally, the mixoviruses, and rhinoviruses such as the corona virus, as well as human immunodeficiency virus ("HIV"), are of the RNA types, while viruses such as the papovaviruses (herpes), the adenoviruses (pox) and the orthomyxoviridie (influenza and parainfluenza) are DNA based. Corona viruses, such as those that cause the common cold, are known to infect a wide variety of hosts. For example, corona viruses are known to infect humans, dogs, cats, pigs and birds.

Virucides play a critical role in limiting or irradicating the deleterious presence of pathogenic viruses in a wide variety of settings, such as in hospitals, patient rooms, treatment facilities, and other surfaces in which the inactivation of viruses would have beneficial effects. Nevertheless, the need for potent and effective virucides must be counterbalanced against concerns for environmental safety.

Cetylpyridinium chloride (CPC) is a quaternary ammonium compound as described in The Merck Index (The Merck Index, 11th Ed.; Merck & Co., Rahway, New Jersey (1989); p. 311) with antiseptic and preservative properties. It is a cationic compound that is presently known to have broad based antibacterial properties, thus making it an active ingredient for the inactivation of both gram negative and gram positive bacteria. Examples of bacteria that are known to be susceptible are: Escherichia coli, Salmonella typhimurium, Pseudomonas aeruginosa, Bacillus anthracis, Bacillus subtilis, Campylobacter, Listeria, Staphylococcus aureus, and Streptococcus pneumoniae. Cetylpyridinium chloride is also known for its antifungal properties affecting such fungi as Candida albicans and spp., Saccharomyces cerevisiae, Torulopsis glabrata, Trichophyton sp., Aspergillus flavus and niger, Stachybotrys atra, Chaetomium globosum, Histoplasma capsulatum, Penicillium cyclopium, and Cladosporum resinae (Reed, R.H ., "Cetyl Pyridinium Chloride: Status Report." University of Northumbria School of Applied and Molecular Biology, January 2002).

The recognized broad spectrum antimicrobial antiseptic effect of CPC without disturbance of intra-oral bacterial flora has resulted in its common use in oral rinses and lozenges. Commercial oral rinse products include, for example, Scope® (Procter and Gamble), Cepacol® (J.B. Williams), and Act® (Johnson & Johnson). Formulation concentrations of CPC in these products range in the 0.04% to 0.05% and are generally recognized as safe levels while possessing the ability to be effective against undesired microbes. There are also multiple citations of CPC efficacy as an active ingredient in the reduction and inhibition of plaque and gingivitis, thus encouraging its use as a dental hygiene constituent (Hunter-Rinderele et al., J. Clin. Res. 72:107113, 1997). However, much evidence exists wherein the efficacy of CPC as an antimicrobial is severely hindered by commonly used ingredients in the formulations of rinses, lozenges, dentifrices and oral care products (Addy et al., J. Dent. Res. 72:719, 1993). Other commercial antiseptic product applications include feminine washes and topical antiseptics for abrasions and minor wounds.

Elimination of viral pathogens, especially those existing upon inanimate surfaces, where such pathogens may remain active for extended periods of time, has been a long standing challenge to maintaining an antiseptic environment in a wide variety of settings. A number of disinfectants have been developed in attempts to provide an adequate mechanism for the elimination of such statically existing viral pathogens. However, many of these disinfectants tend to have less desirable characteristics. For example, some may be corrosive while others may be repugnant or cause discoloration of a treated area.

What is needed, therefore, is a virucidal agent that provides for surface contact disinfection and a composition for the medical treatment and prevention of infection caused by viral particles, while minimizing or eliminating the adverse effects that may be experienced with conventional agents.

The present invention is directed to overcoming, or at least reducing the effects of, one or more of the problems set forth above.

### SUMMARY OF THE INVENTION

The present invention includes compositions and methods for use in inactivating viruses residing on inanimate objects; within the respiratory tract of humans or other animals, especially the oral or nasal cavities; and with the anal or urogenital tract of humans for the prevention or inhibition of HIV. The present invention also includes methods and compositions for decontaminating areas, tools or devices infected by viral pathogens such as those commonly found in hospitals, public restrooms, transportation vehicles, and institutions where mass population would require sanitization of surfaces with maximum residual results.

The present invention includes the use of solubilized CPC in a liquid or other suitable media. Certain embodiments comprise solubilizing CPC in either potable or sterile water for use as a surface contact disinfectant or as a spray in oropharyngeal applications for the destruction of viral agents in the respiratory tracts of humans or other animals. While, still other embodiments of the present invention provide a virucidal kit comprising a CPC containing compound. In such kits, the CPC containing compound may be provided in a concentrated form for dilution to a working concentration prior to use.

Certain other embodiments of the present invention provide compositions that may be safely ingested by humans and other animals, thereby making the compositions and methods of the present invention suitable for treating humans and other animals, exposed to pathogenic viruses. In alternative embodiments, effective amounts of the CPC may be applied to the human and other animal being treated prior to exposure to the pathogenic viruses. Therefore, the present invention includes compositions and methods for use in both the prevention and treatment of viral infections. In these particular embodiments, the present invention may comprise an inactivating composition suitable for pharmaceutical administration to humans and/or other animals. Such compositions suitable for pharmaceutical administration may also comprise one or more pharmaceutically acceptable carriers. These compositions may be applied topically to mucus membranes or oral surfaces including the buccal cavity and throat as a spray or oral rinse (or other acceptable form) or to any other suitable surface of the body, including dermal surfaces and open wounds and the anal or vaginal tract, to treat or prevent viral infections.

### DEFINITIONS

"Inactivate" or "Inactivating," means having the ability to destroy, eliminate or reduce the capacity of a viral pathogen to infect and/or cause a pathological response in a host; wherein such inactivating ability preferably provides at least a one-log order reduction (90%) in the viral pathogens ability to infect and/or cause a pathological response in a host, more preferably a two-log order reduction (99%), and most preferably a three-log order reduction (99.9%).

"Contact" or "contacted" refers to bringing one or more of the compositions of the present invention into contact with a viral pathogen such that the composition(s) of the present invention may inactivate the viral pathogens.

"Enhancers" describes compounds or substances that act to enhance or accelerate the ability of CPC to inactivate viral pathogens.

"Extenders" describes compounds or substances that act to extend or prolong the inactivating ability of CPC on viral pathogens.

"Pharmaceutically acceptable" refers to compositions that do not produce significant adverse reactions when administered or applied to a particular host (e.g., a human or other animal).

"Pharmaceutically acceptable carrier" includes any and all solvents, media, coating or wetting agents, and the like, as well as certain enhancers and extenders, which when utilized remain pharmaceutically acceptable.

### DETAILED DESCRIPTION OF SPECIFIC EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developer's specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

The present invention provides for compositions of solubilized CPC in a liquid or other suitable media and the use of these compositions to inactive viral pathogens according to the claims. The present invention further comprises solubilizing CPC in any suitable liquid media, such as potable or sterile water or an isotonic saline solution, for uses as a surface contact disinfectant or as a spray in applications for the destruction of viral agents in the respiratory tracts of humans or other animals. Solubilizing CPC should be done by adding selected amounts of CPC powder into the liquid media choice. Alternative embodiments of the compositions of the present invention also include the addition of a stabilizing agent, such as polypropylene glycol, to stabilize and reduce the freezing point of the resulting solution.

### 1. Surface Contact Disinfectant

In embodiments of the present invention, which are directed toward surface contact disinfectants and disinfection, the CPC solution concentrations may range from greater than 0.0% up to around 1.0%, the concentrations varying as to specific use. In certain embodiments directed toward household applications, a solution concentration of greater than 0.0% to 1.0%, preferably from 0.01% to 0.5%, more preferably from 0.02% to 0.05%, most preferably around 0.025%, would provide a benign but effective antibacterial disinfectant that would additionally promote the inactivation of certain viral agents, including the corona virus and the influenza virus and the HIV virus. In applications requiring more aggressive disinfection where more virulent strains of viral organisms exist, higher concentrations of CPC up to around 1.0% may be required to assure maximal contact destruction of contaminants on surfaces with residual presence to inhibit further contamination within a reasonable period of time.

CPC also possesses a surfactant property and aggressively adheres to solid surfaces thus promoting a residual action. Extenders, such as cellulose ethers, including but not limited to hydroxypropyl methylcellulose, or sodium carboxymethylcellulose, may be used in various concentrations as adjuncts in certain embodiments of the present invention for extending or enhancing the toxicity and longevity of residual inactivating effect of CPC. These compounds are widely recognized as viscosity modifiers, emulsifiers, protective colloids in emulsions, and as film forming agents. Viscosity properties are dependent on the degree of substitution as a water soluble polymer. For example, in the case of hydroxypropyl methylcellulose, a substitution of 0.01% to 2.5% will cause film-forming and encapsulation, while 0.5% to 5.0% of carboxymethylcellulose will accomplish similar results. In certain embodiments of the present invention these extender compounds may increase surface attachment and upon drying may result in a solid film with varying degrees of water resistance. In certain other embodiments, it is contemplated that the extender is a non-ionic detergent. Non-ionic detergents that find use in the present invention include, but are not limited to varying concentrations, which would be readily ascertainably to one of skill in the art, of Tween 80, Triton, Tyloxapol, Pluronic and Span.

Other embodiments of the present invention may comprise the addition of enhancers which function to accelerate the inactivating properties of CPC. Alternative embodiments of the compositions of the present invention may comprise solutions containing zinc or copper compounds, which include but are not limited to zinc oxide, zinc acetate, zinc bacitracin, zinc carbonate, zinc citrate, zinc iodate, zinc iodide, zinc peroxide, zinc propionate, zinc stearate, zinc salicylate, cuprous iodide, and cupric oleate. The embodiments of the present invention contain citric acid to act as an enhancer or accelerator. It is believed that the addition of the COOH molecules in the dicarboxylic acid increases the reactivity of the CPC long organic tail. The use of citric acid as an enhancer is of particular efficacy in uses directed toward orthomyxoviridie. In fact, of the coronavirus, RSV virus, orthomyxoviridie, and the retrovirus, only the orthomyxoviridie (influenza and parainfluenza) requires the citric acid addition to be effective.

The concentrations necessary will vary from enhancer to enhancer but would be readily ascertainable to one of skill in the art. For example zinc oxide may be used as an enhancer at concentrations of greater than 0.0% weight per volume to as high as around 2.5% weight per volume. Citric acid is used at concentrations ranging from greater than 0.0% weight per volume to as high as around 0.5% weight per volume.

The compositions and methods of the present invention also contemplate combining the CPC compositions with various cleansing applicators, such as wipes, mop heads, sprays, and the like.

The methods of the present invention comprise contacting CPC containing compositions for a time sufficient to inactivate the pathogenic agent or to inhibit the growth of the agent. Contact times sufficient for the inactivation of pathogenic agents may vary from agent to agent but will be readily ascertainable to one of skill in the art. Time periods will preferably range from greater than 0 seconds to around 3 minutes, more preferably from 30 seconds to 1 minute, most preferably a sufficient time period would be around 45 seconds. In other embodiments, the present invention provides a method of decontaminating an environmental surface harboring harmful or undesired pathogens. In one such embodiment, the pathogenic agent is associated with an environmental surface and the method comprises contacting the environmental surface with an amount of the composition sufficient for decontaminating the surface. While it may be so desired, decontamination need not result in total elimination of the pathogen, preferably decontamination will result in a least a one-log reduction (90%) in the presence of active viral pathogens, more preferably a two-log reduction (99%), most preferably a three-log reduction (99.9%). In some embodiments, the compositions and methods further comprise dyes, paints, and other marking and identification compounds as to ensure that a treated surface has been sufficiently treated with the compositions of the present invention.

The present invention further provides methods for protecting (e.g., protecting from contamination of a microorganism) or decontaminating an area (e.g., decontaminating an area by inactivating viral particles in the area) comprising exposing the area to a CPC containing composition comprising an extender and/or enhancer. The exposure times for such areas may vary from area to area and from extender and/or enhancer to extender and/or enhancer but will be readily ascertainably one of skill in the art. Such exposure times will preferably range from greater than 0 seconds to around 3 minutes, more preferably from 30 seconds to 1 minute, most preferably a sufficient time period would be around 45 seconds. These methods may be applied to any type of area or item (e.g., a medical device or treatment table or paper/cloth diapers or mats). For example, in some embodiments, the area may be any variety of hard or soft surfaces, whether porous or non-porous.

Other embodiments of the present invention provide a virucidal kit comprising a CPC containing compound. In such kits, the CPC containing compound may be provided in a concentrated form for dilution to a working concentration prior to use. These kits may also contain one or more extenders and or enhancers. Furthermore, these kits may provide instructions as to a method of use and or desired dilution ratios to be employed. In some embodiments such kits may provide methodologies for targeting particular types of viral pathogens, which may comprise variations in CPC concentrations, or the use of particular extenders and/or enhancers.

### 2. Pharmaceutical Applications

The present invention also comprises compositions and methods for use in the treatment and prevention of virally mediated pathogenic responses in humans and other animals, according to the claims. Viruses mediating such responses would preferably be viruses infecting the respiratory tract of humans and other animals, especially the upper respiratory tract. For example, suitable CPC containing compounds are effective in inactivating the corona virus, which is capable of infecting a wide variety of hosts including humans, dogs, cats, pigs and birds. CPC containing compounds are also capable of inactivating orthomyxoviridie or influenza viruses. The compositions and methods of the present invention will also be useful in the treatment and prevention of virally mediated pathogenic responses on dermal surfaces and open wounds in humans and other animals. For example, in the treatment and prevention of pathogenic states which result from dermal contact with anthrax. Furthermore, the CPC containing compounds can inactivate the HIV. Therefore, in certain embodiments, the present invention may be useful in the treatment and prevention of sexually transmitted diseases, such as HIV, via application to the anal or urogenital tracts in the form of a foam, film, liquid or suppository.

In some embodiments, the present invention provides compositions for use in treating humans and other animals, exposed to pathogen viruses. In alternative embodiments, effective amounts of the CPC are applied to the human and other animal being treated prior to exposure to the viral pathogens. Therefore, the present invention comprises compositions and methods for both the prevention and treatment of viral infections. In these particular embodiments, the present invention may comprise an inactivating composition suitable for pharmaceutical administration to humans and/or other animals.

In the case of upper respiratory tract infections, such as the flu or the common cold, the viral infection remains primarily located in the throat and respiratory tract and causes a susceptibility to infection by other microbial organisms by weakening tissues and inviting secondary bacterial infections. In the present invention, delivery of CPC to these areas via a spray, such as by a pump device or a propelled aerosol, would enable contact destruction of the virus as well as secondary infectious bacterial agents sensitive to CPC. Such devices are readily available in the marketplace and include metered dose inhalants by Cambridge Consultants or Wilden. Pharmaceutically acceptable CPC containing compositions may be utilized in methods of the present invention to directly target tissue areas of infection within the host. For example, an oropharyngeal spray or rinse may be used to deliver suitable CPC containing composition to the throat, buccal cavity and/or nasal passages of an infected individual in concentrations which would be effective in inactivating the viral pathogens. Furthermore, such a spray or rinse may even be used to inactivate viral pathogens upon suspected exposure prior to actual infection.

In the case of an oropharyngeal application, CPC in the range of greater than 0.0% to 0.5%, preferably 0.01% to 0.1%, more preferably 0.025% to 0.05%, in sterile solution would be applied as a spray to the area of the buccal cavity and throat to enable contact destruction of viral agents of the mucosal surfaces to reduce or eliminate infection of viral agents. The compositions and methods of the present invention are also useful in the treatment and prevention of virally mediated pathogenic responses in the lungs of humans and other animals. In the case of the treatment and prevention of viral pathogens in the lungs of humans and other animals, CPC containing pharmaceutically acceptable compositions may be applied as a mist or mist-like inhalant for direct contact with the tissues of the bronchial tract and lung alveoli and surrounding tissue. In such intra-lung applications, useful CPC concentrations in sterile solution may be in the range of greater than 0.0% to 0.5% weight per volume, preferably 0.01 % to 0.1 %, more preferably 0.025% to 0.05%. Additional affectation of viral infection may occur as the transport of the CPC solution ensues within cellular structures of tissue hosting viral agents. Since CPC is highly cationic, it is readily adsorbed and retained on oral and mucosal surfaces, providing a mechanism for extended residual action.

Such compositions suitable for pharmaceutical administration may also comprise one or more pharmaceutically acceptable carriers. These compositions can be applied topically to mucous membranes or oral surfaces including the buccal cavity and throat as a spray or oral rinse (or other acceptable form) or to any other suitable surface of the body, including dermal surfaces and open wounds, to treat or prevent viral infections.

Certain embodiments of the present invention also provide compositions and methods for the treatment and prevention of sexually transmitted diseases, particularly HIV. The CPC containing compounds may be provided in the form of a gel, foam, film, liquid or suppository. In such forms, the compounds may be applied directly to the anal or urogenital tracts of an infected individual. Alternatively, the compounds of the present invention may be useful in indirect applications such as an additive in the packaging of condoms and other non-pharmaceutical birth control devices.

Extenders may be used in compositions of the present invention in addition to or as pharmaceutically acceptable carries. Such extenders include but are not limited to the cellulose ethers hydroxypropyl methylcellulose or sodium carboxymethylcellulose which may be used in various concentrations. In certain other embodiments, it is contemplated that the extender is a non-ionic detergent. Non-ionic detergents that fmd use in the present invention include, but are not limited to varying concentrations, which would be readily ascertainable to one of skill in the art, of Tween 80, Triton, Tyloxapol, Pluronic and Span.

Alternate embodiments of the present invention may contain suitable enhancers to accelerate the inactivating properties of CPC. For example, zinc, in the form of zinc oxide, may be included in the CPC containing compositions to accelerate the CPC mediated inactivation of viral particles. The composition contains citric acid to act as an enhancer or accelerator. The use of citric acid as an enhancer is of particular efficacy in uses directed toward orthomyxoviridie. In still other embodiments of the present invention CPC containing compositions may contain both extenders and enhancers.

The concentrations necessary will vary from enhancer to enhancer but would be readily ascertainable to one of skill in the art. For example zinc oxide may be used as an enhancer at concentrations of greater than 0.0% weight per volume to as high as around 2.5% weight per volume. Citric acid is used at concentrations ranging from greater than 0.0% weight per volume to as high as around 0.5% weight per volume.

Additionally, in still other embodiments of the present invention, the formulations further comprise any number or variation of coloring or flavoring agents (e.g., dyes and peppermint oil).

While the invention has been described with particular reference to specific embodiments thereof, it is to be understood that the forms of the invention shown and described in detail are to be taken as preferred embodiments thereof. Various changes and modifications may be resorted to without departing from the spirit and scope of the invention as defined by the claims.

### EXAMPLES

The present invention derives from in vivo trials and challenge studies (1) in determining whether CPC may be effective as an antiviral agent and, if effective, the minimum inhibitory dosage; (2) in determining any adverse effects of the host cells and dosages thereto; and, (3) in comparing antiviral effects of CPC, benzalkonium chloride, and triclosan. As an exemplary embodiment, the present invention entails a method to directly measure the extent to which a potential antiviral substance inhibits the effects of viral infection in a tissue culture. Plaque reduction is recommended for virus and cell line combinations which produce a well-defined plaque. Other cytopathic effects (CPE) can be measured in those virus and cell lines which do not produce well-defined plaques, or where a specific cytopathic effect may be a defining characteristic.

### Example 1: Corona Virus.

Feline Infectious Peritonitis Type 2 (FIP Type 2) corona virus cultures were grown in CrFK cell culture in order to demonstrate plaque formation and the effect of CPC compounds of the present invention on Corona Virus activity. Untreated virus served as controls. Alternatively, virus suspensions were exposed to three concentrations of CPC for 300 seconds. The concentrations were produced by adding certified USP grade cetylpyridinium chloride powder to sterile water to produce 10ml each of solution concentrations at 0.025%, 0.05%, and 0.10%. At the end of the exposure, the virus was titrated in CrFK cell culture. Viral titration is accomplished at each test article concentration by seeding 0.1 ml of a serial dilution of treated virus to the CrFK cell culture. Serial dilutions are prepared from 10⁻¹ to 10⁻⁸ of the original virus concentration, or five million/ml (6.7 log units) and each dilution then placed in each of four wells (replicates) of a 96 well microplate. The inoculated cells (80 µl per well) were then incubated at 37° C in a CO₂ incubator for five days. They were then observed microscopically for CPE inhibition, reporting titers as TCID50 /ml, also expressed as plaque forming units (pfu's). The corrected virus titer (# positives x dilution) is reported in log/ml. Virucidal activity is thus defined as the number of logs reduction of virus titer between the test article and the control (Reed and Meunch). The following table demonstrates the efficacy of CPC in the destruction of FIP Type2 corona virus:

| **Bioassay of FIP-2 Corona Virus against CPC** | | | |
|---|---|---|---|
| **CPC %** | **FIP-2 Titer** | **Reduction in Titer** | **% Reduction** |
| | | | |
| 0.10 | < 3.50 logs/ml * | > 3.20 logs | 99.97 |
| 0.05 | < 3.50 logs/ml * | > 3.20 logs | 99.97 |
| 0.025% | 3.30 logs/ml | 3.40 logs | 99.95 |
| Control | 6.70 logs/ml | N/A | N/A |

| | | | |
|---|---|---|---|
| *Dilutions of 10⁻² and below at CPC concentrations of 0.10% and 0.05% did demonstrate cell toxicity and could not be read with certainty. No virus were observed. Note: 6.7 log units = 5,000,000 virus particles; 3.30 log units = ∼ 2,100 virus particles. | | | |

In the present invention, a significant reduction in virus titer occurred as a result of a five minute exposure of a viral suspension containing five million virus/ml in the presence of a 0.025% concentration of CPC. This reduction transposes into a reduction of greater than 99.95% of viral particles present. Assuming the same killing effect on a surface area, the application of CPC at a concentration of 0.025%/in² would result in a greater than 3-log reduction of virus. Comparative testing with benzalkonium chloride and triclosan as substitute active ingredients in the three concentrations as employed with CPC produced disparate results and demonstrated increased cellular toxicity. With benzalkonium chloride, only the 0.10% exposure resulted in viral reduction of which a percentage reduction was approximately 20%. Triclosan demonstrated cellular toxicity at all three levels with no viral reduction at all three levels. In the present invention, CPC at a concentration of 0.025% in solution consistently demonstrated a greater than 3-log lethal toxicity of the FIP Type2 corona virus for five minutes without adverse cytopathic effects.

### Example 2: Orthomyxoviridie.

Orthomyxoviridie virus cultures were grown in MDCK (NBL-2)5 (ATCC type CCL-34) cell culture in order to demonstrate plaque formation and the effect of CPC compounds of the present invention on influenza type viral activity. Influenza virus, specimen Influenza A/Equi 2?Miami 1/63, ATCC VR517, was passaged in cell culture to provide viral particles sufficient for conducting the in vitro assays. Serial dilutions were prepared from 10⁻¹ to 10⁻⁸ of the original virus concentration, or five million/ml (6.7 log units) and each dilution then placed in each of four wells (replicates) of a 96 well microplate. Control titers averaged 6.5 logs/ml. Virus suspensions were exposed to three concentrations of CPC for 300 seconds. The inoculated cells (80 µl per well) were then incubated at 37° C in a CO₂ incubator for five days. Untreated virus served as a base line control.

Upon introduction of 0.05% and 0.025% of CPC, no significant reduction was observed in the titer after a five minute exposure. In addition, subsequent tests involving treatment with TSP and acetic acid at 0.1% addition to the CPC for purposes of pH adjustment showed no significant impact on the virus titer.

The addition of 0.1% citric acid (weight/volume) to both the 0.025% (weight/volume) and 0.05% (weight/volume) CPC solutions, however, resulted in a greater than 3.5 log reduction to the virus titers. Furthermore, the use of test solutions containing 0.025% CPC and 0.1% citric acid caused no observed cytotoxic affects. Additional testing in the presence of sera also resulted in the same log reduction. Furthermore, the pH of both the citric and acetic acid at 0.1% was 2.3 and neither solution was cytotoxic at those levels. All results were based upon the Reed and Muench method.

### Example 3: FeLV.

FeLV retrovirus cultures were grown in CrFK (ATCC type CCL-94) cell culture in order to demonstrate plaque formation and the effect of CPC compounds of the present invention on the feline surrogate to HIV. FeLV retrovirus was gown in cell culture to a titer of 6.0 logs/ml. Initial testing for cytotoxicity resulted in data consistent with the corona virus testing.

Briefly, virus suspensions were exposed to three concentrations of CPC, 0.025%, 0.05%, and 0.10%, for 300 seconds. Untreated virus served as controls. At the end of the exposure, the virus was titrated in CrFK cell culture. Viral titration was accomplished at each test article concentration by seeding 0.1 ml of a serial dilution of treated virus to the CrFK cell culture. Serial dilutions were prepared from 10⁻¹ to 10⁻⁸ of the original virus concentration, or five million/ml (6.7 log units) and each dilution then placed in each of four wells (replicates) of a 96 well microplate. The inoculated cells (80 µl per well) are then incubated at 37° C in a CO₂ incubator for five days. CPC treatment at a concentration of 0.025% was not cytotoxic, while concentrations of 0.05% and 0.1% were.

CPC at a CPC level of 0.025%, both in conjunction 0.1% citric acid and without it, caused a greater than 3.5 logs/ml reduction in the viral titer. The presence or absence of sera had no effect on the CPC mediated inactivation of the FeLV virus. Furthermore, exposure of the virus to all three levels of CPC with or without citric acid at 0.1% resulted in a 100% reduction of the FeLV virus. All results were based upon the Reed and Muench method.

### Example Summary.

It would be recognized by those in the art that the FIP-2 corona and orthomyxoviridie viruses represent an enduring and resilient viral specimen with high resistance to antibiotics and biocides. Corona is an enveloped virus, closely resembling the SARS virus, and is a surrogate for laboratory testing of the SARS virus. The corona and orthomyxoviridie viruses are hardy organisms and have an ability to reside outside of host systems for extended periods of time. These viruses have a resistance to drying, according to types, and may reside on surfaces for extended periods with full ability to transfer to a host and cause infection.

Based upon the studies of the present invention, application of a 0.025% CPC solution to solid surfaces via a spray device or wipe or direct liquid pour would result in an effective disinfectant application and eliminate the corona and orthomyxoviridie viruses. Additionally, since the studies substantiate no adverse cellular affectation at the 0.025% solution level, the introduction of the certain compositions of the present invention to the buccal cavity and throat area in humans may provide a benign but effective antiseptic and inhibitor of the corona and orthomyxoviridie viruses and other less hardy viruses. In addition, as the prior art will further support the antibiotic and antifungal properties associated with CPC, it would provide another benefit in the use of the compound for treatment of viral maladies such as the common cold, flu, or SARS. Specifically, the addition of a 0.025% CPC solution to mucosal surfaces of the respiratory tract would benefit the infected individual by inhibiting secondary infections associated with bacteria.

The particular embodiments disclosed above are illustrative only, as the invention may be modified and practiced in different but equivalent manners apparent to those skilled in the art having the benefit of the teachings herein. Furthermore, no limitations are intended to the details of construction or design herein shown, other than as described in the claims below. It is therefore evident that the particular embodiments disclosed above may be altered or modified and all such variations are considered within the scope of the invention. Accordingly, the protection sought herein is as set forth in the claims below.

## Claims

1. A non-therapeutic method for inactivating viral pathogens comprising:
(a) providing a virucidal composition comprising a liquid media containing citric acid and less than 1 % weight per volume cetylpyridinium chloride; and
(b) contacting the virucidal composition with a surface targeted for disinfection.

2. The method of claim 1, wherein the liquid media is selected from the group consisting of: sterile water, potable water and sterile saline.

3. The method of claim 1 wherein the surface targeted for disinfection is an inanimate surface.

4. The method of claim 1 or 3, wherein the virucidal composition comprises less than 0.5% weight per volume cetylpyridinium chloride.

5. The method of claim 1 or 3, wherein the virucidal composition comprises less than 0.05% weight per volume cetylpyridinium chloride.

6. The method of claim 1 or 3, wherein the virucidal composition further comprises at least one extender.

7. The method of claim 1 or 3, wherein the virucidal composition further comprises at least one enhancer and at least one extender.

8. The method of claim 7, wherein the at least one extender is a cellulose ether.

9. The method of claim 1 or 3, wherein the virucidal composition is contacted with the surface targeted for disinfection through the use of an applicator.

10. The method of claim 1 or 3, wherein the virucidal composition further comprises a scented component.

11. The method of claim 1 or 3, wherein the virucidal composition is contacted to the surface for one of at least 45 seconds or at least 3 minutes.

12. A virucidal composition comprising:
(a) a liquid media;
(b) cetylpyridinium chloride in solution in the liquid media at a concentration of less than 1% weight per volume;
(c) citric acid;
(d) an extender; and
(e) an enhancer.

13. A virucidal composition according to claim 12 for use in the treatment of virulent infections.

14. The composition of claim 13, wherein the virucidal composition is in a form selected from the group consisting of an oral rinse, a gel, a foam, a film, a mist, and a suppository.

15. A condom coated with the virucidal composition of claim 12.

16. The composition or use of claim 13, wherein the virulent infection is in the lungs, the urogenital tract, or the anal tract of an organism.

17. The composition of claim 13 wherein the infected area is a mucous membrane.

18. The composition of claim 17, wherein the mucous membrane is selected from the group consisting of: in the nasal cavity; a buccal cavity, an oropharyngeal cavity, a urogenital tract and an anal tract.

19. The composition of claim 12, wherein the virucidal composition comprises less than 0.5% weight per volume cetylpyridinium chloride.

20. The composition of claim 12, wherein the virucidal composition comprises less than 0.05% weight per volume cetylpyridinium chloride.

21. The composition of claim 12, wherein the virucidal composition comprises less than 0.03% weight per volume cetylpyridinium chloride.

22. The composition of claim 12, wherein the extender is a cellulose ether.

23. The composition of claim 22, wherein the cellulose ether is hydroxypropyl methylcellulose or sodium carboxymethylcellulose.

24. The composition of claim 12, wherein the extender is a non-ionic detergent.

25. The composition of claim 24, wherein the non-ionic detergent is selected from the group consisting of: Tween 80, Triton, Tyloxapol, Pluronic and Span.

26. The composition of claim 25, wherein the enhancer is selected from a group consisting of: zinc oxide, zinc acetate, zinc bacitracin, zinc carbonate, zinc citrate, zinc iodate, zinc iodide, zinc peroxide, zinc propionate, zinc stearate, zinc salicylate, cuprous iodide, and cupric oleate.

27. The composition of claim 12, wherein the citric acid is at a concentration of between greater than 0.0% and 0.5% weight per volume.

28. The composition of claim 12, wherein the liquid media is selected from the group consisting of: sterile water, potable water, sterile saline or fatty alcohols.

29. The composition of claim 12, wherein the composition is pharmaceutically acceptable.

30. A virucidal composition according to claim 12 for use in inactivation of a virus in a host organism.

## Patentansprüche

1. Nicht therapeutisches Verfahren zum Inaktivieren viraler Krankheitserreger, umfassend:
(a) Bereitstellen einer viruziden Zubereitung, umfassend ein flüssiges Medium, umfassend Zitronensäure und weniger als 1 Gewichtsprozent pro Volumen Cetylpyridiniumchlorid; und
(b) Inkontaktbringen der viruziden Zubereitung mit einer Fläche, die zur Desinfektion markiert ist.

2. Verfahren nach Anspruch 1, wobei das flüssige Medium aus der Gruppe ausgewählt ist, bestehend aus: sterilem Wasser, Trinkwasser und steriler Kochsalzlösung.

3. Verfahren nach Anspruch 1, wobei die Fläche, die zur Desinfektion markiert ist, eine unbelebte Fläche ist.

4. Verfahren nach Anspruch 1 oder 3, wobei die viruzide Zubereitung weniger als 0,5 Gewichtsprozent pro Volumen Cetylpyridiniumchlorid umfasst.

5. Verfahren nach Anspruch 1 oder 3, wobei die viruzide Zubereitung weniger als 0,05 Gewichtsprozent pro Volumen Cetylpyridiniumchlorid umfasst.

6. Verfahren nach Anspruch 1 oder 3, wobei die viruzide Zubereitung überdies mindestens ein Streckmittel umfasst.

7. Verfahren nach Anspruch 1 oder 3, wobei die viruzide Zubereitung überdies mindestens einen Geschmacksverstärker und ein Streckmittel umfasst.

8. Verfahren nach Anspruch 7, wobei das mindestens eine Streckmittel ein Celluloseether ist.

9. Verfahren nach Anspruch 1 oder 3, wobei die viruzide Zubereitung mit der Fläche, die zur Desinfektion markiert ist, durch die Verwendung eines Applikators in Kontakt gebracht wird.

10. Verfahren nach Anspruch 1 oder 3, wobei die viruzide Zubereitung überdies eine wohlriechende Komponente umfasst.

11. Verfahren nach Anspruch 1 oder 3, wobei die viruzide Zubereitung über mindestens 45 Sekunden oder mindestens 3 Minuten mit der Fläche in Kontakt gebracht wird.

12. Viruzide Zubereitung, umfassend:
(a) ein flüssiges Medium;
(b) Cetylpyridiniumchlorid in Lösung in dem flüssigen Medium bei einer Konzentration von weniger als 1 Gewichtsprozent pro Volumen;
(c) Zitronensäure;
(d) ein Streckmittel; und
(e) einen Geschmacksverstärker

13. Viruzide Zubereitung nach Anspruch 12 zur Verwendung bei der Behandlung virulenter Infektionen.

14. Zubereitung nach Anspruch 13, wobei die viruzide Zubereitung in einer Form vorliegt, die aus der Gruppe ausgewählt ist, bestehend aus einer Mundspüllösung, einem Gel, einem Schaum, einem Film, einem Nebel und einem Suppositorium.

15. Kondom, das mit der viruziden Zubereitung nach Anspruch 12 beschichtet ist.

16. Zubereitung oder Verwendung nach Anspruch 13, wobei sich die virulente Infektion in den Lungen, dem Urogenitaltrakt oder dem Analtrakt eines Organismus' befindet.

17. Zubereitung nach Anspruch 13, wobei der infizierte Bereich eine Schleimhaut ist.

18. Zubereitung nach Anspruch 17, wobei die Schleimhaut aus der Gruppe ausgewählt ist, bestehend aus: in der Nasenhöhle; einer Mundhöhle, einer Mundrachenhöhle, einem Urogenitaltrakt und einem Analtrakt.

19. Zubereitung nach Anspruch 12, wobei die viruzide Zubereitung weniger als 0,5 Gewichtsprozent pro Volumen Cetylpyridiniumchlorid umfasst.

20. Zubereitung nach Anspruch 12, wobei die viruzide Zubereitung weniger als 0,05 Gewichtsprozent pro Volumen Cetylpyridiniumchlorid umfasst.

21. Zubereitung nach Anspruch 12, wobei die viruzide Zubereitung weniger als 0,03 Gewichtsprozent pro Volumen Cetylpyridiniumchlorid umfasst.

22. Zubereitung nach Anspruch 12, wobei das Streckmittel ein Celluloseether ist.

23. Zubereitung nach Anspruch 23, wobei der Celluloseether Hydroxypropylmethylzellulose oder Natriumcarboxymethylcellulose ist.

24. Zubereitung nach Anspruch 12, wobei das Streckmittel ein nichtionogenes Tensid ist.

25. Zubereitung nach Anspruch 24, wobei das nichtionogene Tensid aus der Gruppe ausgewählt ist, bestehend aus: Tween 80, Triton, Tyloxapol, Pluronic und Span.

26. Zubereitung nach Anspruch 25, wobei das Streckmittel aus einer Gruppe ausgewählt ist, bestehend aus: Zinkoxid, Zinkacetat, Zinkbacitracin, Zinkcarbonat, Zinkcitrat, Zinkiodat, Zinkiodid, Zinkperoxid, Zinkpropionat, Zinkstearat, Zinksalicylat, Kupferiodid und Kupferoleat.

27. Zubereitung nach Anspruch 12, wobei die Zitronensäure eine Konzentration hat, die zwischen größer als 0,0 und 0,5 Gewichtsprozent pro Volumen ist.

28. Zubereitung nach Anspruch 12, wobei das flüssige Medium aus der Gruppe ausgewählt ist, bestehend aus: sterilem Wasser, Trinkwasser, steriler Kochsalzlösung oder Fettalkoholen.

29. Zubereitung nach Anspruch 12, wobei die Zubereitung pharmazeutisch annehmbar ist.

30. Viruzide Zubereitung nach Anspruch 12 zur Verwendung bei der Inaktivierung eines Virus' in einem Wirtsorganismus.

## Revendications

1. Procédé non thérapeutique pour inactiver des pathogènes viraux comprenant :
(a) la production d'une composition virucide comprenant un milieu liquide contenant de l'acide citrique et moins de 1 % en poids par volume de chlorure de cétylpyridinium ; et
(b) la mise en contact de la composition virucide avec une surface ciblée pour désinfection.

2. Procédé de la revendication 1, **caractérisé en ce que** le milieu liquide est choisi dans le groupe constitué de : l'eau stérile, l'eau potable et le soluté stérile.

3. Procédé de la revendication 1 **caractérisé en ce que** la surface ciblée pour désinfection est une surface inanimée.

4. Procédé de la revendication 1 ou 3, **caractérisé en ce que** la composition virucide comprend moins de 0,5 % en poids par volume de chlorure de cétylpyridinium.

5. Procédé de la revendication 1 ou 3, **caractérisé en ce que** la composition virucide comprend moins de 0,05 % en poids par volume de chlorure de cétylpyridinium.

6. Procédé de la revendication 1 ou 3, **caractérisé en ce que** la composition virucide comprend en outre au moins un extendeur.

7. Procédé de la revendication 1 ou 3, **caractérisé en ce que** la composition virucide comprend en outre au moins un adjuvant et au moins un extenseur.

8. Procédé de la revendication 7, **caractérisé en ce que** l'au moins un extendeur est un éther de cellulose.

9. Procédé de la revendication 1 ou 3, **caractérisé en ce que** la composition virucide est mise en contact avec la surface ciblée pour désinfection par utilisation d'un applicateur.

10. Procédé de la revendication 1 ou 3, **caractérisé en ce que** la composition virucide comprend en outre un composant parfumé.

11. Procédé de la revendication 1 ou 3, **caractérisé en ce que** la composition virucide est mise en contact avec la surface pendant l'un d'au moins 45 secondes ou au moins 3 minutes.

12. Composition virucide comprenant :
(a) un milieu liquide ;
(b) du chlorure de cétylpyridinium en solution dans le milieu liquide à une concentration de moins de 1 % en poids par volume ;
(c) de l'acide citrique ;
(d) un extendeur ; et
(e) un adjuvant.

13. Composition virucide selon la revendication 12 pour utilisation dans le traitement d'infections virulentes.

14. Composition de la revendication 13, **caractérisée en ce que** la composition virucide est sous une forme choisie dans le groupe constitué d'un rinçage oral, un gel, une mousse, un film, un brouillard et un suppositoire.

15. Préservatif revêtu avec la composition virucide de la revendication 12.

16. Composition ou utilisation de la revendication 13, **caractérisée en ce que** l'infection virulente est dans les poumons, le tractus urogénital ou le tractus anal d'un organisme.

17. Composition de la revendication 13 **caractérisée en ce que** la zone infectée est une membrane muqueuse.

18. Composition de la revendication 17, **caractérisée en ce que** la membrane muqueuse est choisie dans le groupe constitué de : l'intérieur de la cavité nasale ; la cavité buccale, la cavité oropharyngée, le tractus urogénital et le tractus anal.

19. Composition de la revendication 12, **caractérisée en ce que** la composition virucide comprend moins de 0,5 % en poids par volume de chlorure de cétylpyridinium.

20. Composition de la revendication 12, **caractérisée en ce que** la composition virucide comprend moins de 0,05 % en poids par volume de chlorure de cétylpyridinium.

21. Composition de la revendication 12, **caractérisée en ce que** la composition virucide comprend moins de 0,03 % en poids par volume de chlorure de cétylpyridinium.

22. Composition de la revendication 12, **caractérisée en ce que** l'extendeur est un éther de cellulose.

23. Composition de la revendication 22, **caractérisée en ce que** l'éther de cellulose est l'hydroxypropylméthylcellulose ou la carboxyméthylcellulose sodique.

24. Composition de la revendication 12, **caractérisée en ce que** l'extendeur est un détergent non ionique.

25. Composition de la revendication 24, **caractérisée en ce que** le détergent non ionique est choisi dans le groupe constitué de : Tween 80, Triton, Tyloxapol, Pluronic et Span.

26. Composition de la revendication 25, **caractérisée en ce que** l'adjuvant est choisi dans le groupe constitué de : l'oxyde de zinc, l'acétate de zinc, la bacitracine de zinc, le carbonate de zinc, le citrate de zinc, l'iodate de zinc, l'iodure de zinc, le peroxyde de zinc, le propionate de zinc, le stéarate de zinc, le salicylate de zinc, l'iodure cuivreux, et l'oléate cuprique.

27. Composition de la revendication 12, **caractérisée en ce que** l'acide citrique est à une concentration comprise entre plus de 0,0 % et 0,5 % en poids par volume.

28. Composition de la revendication 12, **caractérisée en ce que** le milieu liquide est choisi dans le groupe constitué de : l'eau stérile, l'eau potable, le soluté stérile ou des alcools gras.

29. Composition de la revendication 12, **caractérisée en ce que** la composition est pharmaceutiquement acceptable.

30. Composition virucide selon la revendication 12 pour utilisation dans l'inactivation d'un virus dans un organisme hôte.
